# EUROPÄISCHE PATENTANMELDUNG

(11) **EP 4 268 805 A1**
(43) Veröffentlichungstag der Anmeldung: **01.11.2023**
(21) Anmeldenummer: 22170893.6
(22) Anmeldetag: 29.04.2022
(51) Int. Cl.: A61K 9/19, A61K 31/665

(54) **FOSFOMYCIN-FORMULIERUNG ZUR PARENTERALEN VERABREICHUNG UND HERSTELLUNGSVERFAHREN**

(71) Anmelder: Georgopoulos, Apostolos, 1130 Wien (AT); Schifer, Albert, 1210 Wien (AT)
(72) Erfinder: Georgopoulos, Apostolos, 1130 Wien (AT); Schifer, Albert, 1210 Wien (AT)
(74) Vertreter: SONN Patentanwälte OG

(57) **Zusammenfassung**

Die Erfindung betrifft ein Verfahren zur Herstellung eines geschlossenen Behälters, wobei der Behälter ein Lyophilisat zur Rekonstitution zu einer Lösung zur parenteralen Verabreichung beinhaltet, umfassend die folgenden Schritte: Herstellen einer wässrigen Lösung, wobei in der Lösung zumindest Fosfomycin bzw. ein pharmazeutisch annehmbares Salz davon, vorzugsweise als einziger Wirkstoff, und eine pharmazeutisch annehmbare Säure gelöst sind, Befüllen zumindest eines Behälters mit der wässrigen Lösung, Lyophilisieren der wässrigen Lösung im Behälter und Verschließen des Behälters, wodurch der geschlossene Behälter erhalten wird. Ferner betrifft die Erfindung einen durch dieses Verfahren erhältlichen geschlossenen Behälter, sowie ein Set umfassend diesen Behälter.

## Beschreibung

Das Gebiet der vorliegenden Erfindung ist das der Fosfomycin-Formulierungen zur parenteralen Verabreichung, insbesondere zur intravenösen Verabreichung.

Das Antibiotikum Fosfomycin stellt eine strukturell völlig eigenständige Substanz innerhalb der Antibiotika dar und wurde ursprünglich aus *Streptomyces fradiae* isoliert. Parenteral wird Fosfomycin z.B. intravenös oder intramuskulär verabreicht und wird üblicherweise zur gezielten Therapie von Infektionen insbesondere im intensivmedizinischen Bereich verwendet, vor allem wenn Resistenzen oder Unverträglichkeiten in Bezug auf andere Antibiotika zu erwarten sind. Klinische Anwendungsgebiete für den Einsatz von Fosfomycin sind unter anderem: Im Bereich des zentralen Nervensystems Meningitis, Meningoencephalitis, Hirnabszess und Subduralempyem; in der Chirurgie postoperative Infektionen, Begleitinfektionen bei Tumoren sowie Prostatitis; in der Orthopädie und Traumatologie postoperative Knocheninfektionen (infizierte Osteosynthese, Endoprothese), Osteomyelitis, purulente Arthritis, Abszesse und Phlegmone; im Bereich des Respirationstraktes Bronchopneumonie, Lungenabszess und Nasennebenhöhlenentzündung; sowie Sepsis.

Fosfomycin-Dinatriumsalz ist ein gebräuchliches pharmazeutisch annehmbares Salz von Fosfomycin. Es ist ein weißes oder fast weißes, stark hygroskopisches Pulver, das in Wasser sehr gut löslich ist. In Wasser gelöstes Fosfomycin ist in Folge des hohen pH-Wertes der Lösung parenteral nicht verabreichbar. Um einen gewebeverträglichen bzw. venenverträglichen pH-Wert zu erreichen, wird daher bei Fosfomycinformulierungen zur parenteralen Verabreichung bei zur Zeit verfügbaren Präparaten Bernsteinsäure zugegeben.

Derzeit erfolgt der Zusatz von Bernsteinsäure als Pulver durch sterile Mischung mit Fosfomycin als Pulver. Diese Mischung wird als Pulver in Durchstechflaschen abgefüllt und in dieser Form in den Handel gebracht. In dieser Form als Trockensubstanz zur Infusionsbereitung zugelassen in einzelnen Ländern der Europäischen Union sind z.B. Fosfomycin Astro (Astro-Pharma GmbH, Wien, AT), Fosfomycin medicamentum (medicamenutum pharma GmbH, Allerheilgen, AT) und Fosfomycin Sandoz^{®} (Sandoz GmbH, Kundl, AT) in Österreich, Fosfomycin Infectopharm^{®} (Infectopharm Arzneimittel und Consilium GmbH, Heppenheim, DE) in Deutschland und Fomicyt^{®} (Infectopharm Arzneimittel und Consilium GmbH, Heppenheim, DE) im Vereinigten Königreich.

Direkt vor der parenteralen Verabreichung müssen solche Trockensubstanzen von medizinischem Fachpersonal in einem geeigneten Lösungmittel aufgelöst werden.

Die derzeitige Vorgangsweise verursacht verschiedene Probleme. Die physikalische sterile Mischung von Trockensubstanzen ist sehr aufwändig, z.B. muss die Gleichmäßigkeit der Mischung gewährleistet werden, d.h. es darf nicht zur Entmischung oder zum Zusammenbacken des Pulvers kommen. Hierzu bedarf es einer komplizierten Technologie, die nur wenige Hersteller unter den erforderlichen Good Manufacturing Practice (GMP)-Bedingungen beherrschen. Dadurch kommt es laufend zu Lieferengpässen in der Versorgung mit Fosfomycin zur parenteralen Verabreichung.

Einige dieser Probleme werden auch durch die DE 20 2008 012 514 U1 erkannt. Jenes Dokument schlägt als Ansatz eine Verpackungseinheit zur Bereitstellung einer parenteral verabreichbaren, pharmakologisch akzeptablen pharmazeutischen Formulierung, die das Antibiotikum Fosfomycin und Bernsteinsäure oder eine andere biologisch kompatible Säure enthält, dadurch gekennzeichnet, dass die Einheit ein steril befülltes Behältnis mit in Pulverform vorliegendem Fosfomycin und ein steril befülltes Behältnis mit der in dem pharmakologisch akzeptablen Lösungsmittel gelösten Säure beinhaltet, vor.

Ein anderer Ansatz wird in der WO 2017/158099 A1 vorgeschlagen. Jenes Dokument lehrt, Fosfomycin gemeinsam mit einer pharmazeutisch annehmbaren Säure in Lösung lagerfähig und gebrauchsfertig zur intravenösen Verabreichung zur Verfügung zu stellen.

Trotz dieser Ansätze im Stand der Technik kommt es immer wieder zu Lieferengpässen von Fosfomycin.

Eine Aufgabe der vorliegenden Erfindung ist es nun, eine Fosfomycin-Formulierung zur parenteralen Verabreichung zur Verfügung zu stellen, welche die Nachteile des Standes der Technik wie u.a. Lieferengpässe durch aufwändige Herstellung überwinden soll. D.h. es ist eine erfindungsgemäße Aufgabe, eine Fosfomycin-Formulierung zur parenteralen Verabreichung zur Verfügung zu stellen, die einfacher bzw. zuverlässiger herzustellen ist, bzw. ein entsprechendes Herstellungsverfahren.

Die vorliegende Erfindung stellt ein Verfahren zur Herstellung eines geschlossenen Behälters, wobei der Behälter ein Lyophilisat zur Rekonstitution zu einer Lösung zur parenteralen Verabreichung beinhaltet, zur Verfügung, umfassend die folgenden Schritte:
- Herstellen einer wässrigen Lösung, wobei in der Lösung zumindest Fosfomycin bzw. ein pharmazeutisch annehmbares Salz davon, vorzugsweise als einziger Wirkstoff, und eine pharmazeutisch annehmbare Säure gelöst sind,
- Befüllen zumindest eines Behälters mit der wässrigen Lösung,
- Lyophilisieren der wässrigen Lösung im Behälter und
- Verschließen des Behälters, wodurch der geschlossene Behälter (der das Lyophilisat beinhaltet) erhalten wird. Typischerweise handelt es sich beim Behälter um einen Endverpackungsbehälter (wie z.B. eine Ampulle, Spritze oder Durchstechflasche), d.h. um jenen Behälter, welcher dem medizinischen Fachpersonal zur Verfügung gestellt wird.

In der Lösung können weitere Wirkstoffe und/oder Hilfstoffe enthalten sein. Zweckmäßigerweise ist der Behälter hermetisch (bzw. "hermetisch versiegelt"), d.h. mit anderen Worten, dass kein Gasaustausch zwischen dem darin befindlichen Lyophilisat und der Umgebung des Behälters möglich ist.

In einem weiteren Aspekt betrifft die vorliegende Erfindung einen geschlossenen Behälter, wobei der Behälter ein Lyophilisat zur Rekonstitution zu einer Lösung zur parenteralen Verabreichung beinhaltet, wobei das Lyophilisat Fosfomycin bzw. ein pharmazeutisch annehmbares Salz davon und eine pharmazeutisch annehmbare Säure bzw. ein pharmazeutisch annehmbares Salz davon umfasst. Typischerweise handelt es sich beim Behälter um einen Endverpackungsbehälter (wie z.B. eine Ampulle, Spritze oder Durchstechflasche). Vorzugsweise ist dieser Behälter erhältlich nach dem erfindungsgemäßen Verfahren.

Des weiteren stellt die vorliegende Erfindung ein Set (Verpackungseinheit), umfassend den erfindungsgemäßen geschlossenen Behälter, zur Verfügung. Dieses Set umfasst einen weiteren (geschlossenen) Behälter. Dieser Behälter wiederum beinhaltet (wässriges) Lösungsmittel (insbesondere Wasser zu Injektionszwecken) zur Rekonstitution des Lyophilisats.

In einem letzten Aspekt stellt die vorliegende Erfindung ein Verfahren zur Herstellung einer Lösung zur parenteralen Verabreichung zur Verfügung. Dieses Verfahren umfasst das Öffnen des erfindungsgemäßen geschlossenen Behälters oder das Öffnen des geschlossenen Behälters (der das Lyophilisat beinhaltet) des erfindungsgemäßen Sets, und das Rekonstituieren des Lyophilisats (im Behälter) zur Lösung.

Im Zuge der vorliegenden Erfindung hat sich herausgestellt, dass Lyophilisate, zu deren Herstellung Fosfomycin und Säure (insbesondere Bernsteinsäure, Weinsäure, Milchsäure, Apfelsäure oder Zitronensäure) gemeinsam lyophilisiert worden sind, die hohen Stabilitätsanforderungen für Arzneimittel erfüllen. Durch die Lyophilisierung direkt im (Endverpackungs)behälter wird darüber hinaus verhindert, dass eine mögliche Entmischung der Komponenten des Lyophilisats (vor der Endabfüllung) zur Folge hätte, dass das Mischverhältnis bzw. die Fosfomycin-Dosis nicht mehr den ursprünglich intendierten entspricht. Die vorliegende Erfindung stellt ein zuverlässigeres bzw. einfacheres Herstellungsverfahren für parenterale Fosfomycin-Formulierungen zur Verfügung.

In einer bevorzugten Ausführungsform der vorliegenden Erfindung ist die Lösung (zu der das Lyophilisat durch Gabe des Lösungsmittel rekonstituiert wird) zur Infusion oder Injektion in den Körper eines Säugetiers, insbesondere eines Menschen, vorgesehen. Vorzugsweise ist dieses Säugetier bzw. dieser Mensch ein Patient, der an einer bakteriellen Infektion erkrankt ist.

In einer weiteren bevorzugten Ausführungsform der vorliegenden Erfindung ist die Lösung (zu der das Lyophilisat durch Gabe des Lösungsmittel rekonstituiert wird) zu einer Verabreichung vorgesehen, die ausgewählt ist aus intravenöser Verabreichung, intramuskulärer Verabreichung, intraossärer Verabreichung, intravitrealer Verabreichung, intraperitonealer Verabreichung und intrathekaler Verabreichung. Davon ganz besonders bevorzugt ist die intravenöse Verabreichung.

Vorteilhafterweise wird bzw. ist der erfindungsgemäße geschlossene Behälter (der das Lyophilisat beinhaltet) versiegelt, unter anderem aus Gründen der Manipulationssicherheit bzw. auch um versehentliches Wiederverwenden eines bereits geöffneten und dadurch möglicherweise kontaminierten Behälters zu verhindern. Der Ausdruck "versiegelt" bedeutet im Kontext der vorliegenden Erfindung, dass ein Öffnen des Behälters, das nach dem Versiegeln stattgefunden hat, nachweisbar, bevorzugt sogar mit freiem Auge sichtbar, ist, wobei die besagte Nachweisbarkeit bzw. Sichtbarkeit zumindest nicht ohne Aufwand bzw. ohne erheblichen Aufwand wieder rückgängig gemacht werden kann. Dem Fachmann sind Mittel zur Versiegelung im Arzneimittelbereich bekannt, darunter fallen beispielsweise Abziehfolien, Faserrissetiketten, Void-Siegel, Siegel mit Reißverschlussperforation und Perforation an Drehverschlüssen ("Frischesiegel"). Vorzugsweise erfüllt die Versiegelung des erfindungsgemäßen geschlossenen Behälters bzw. der erfindungsgemäße Behälter die Norm DIN EN 16679:2015-03.

Unter anderem aus ähnlichen Gründen wie im vorigen Absatz genannt ist in einer weiteren bevorzugten Ausführungsform der vorliegenden Erfindung der Behälter in einer Weise geschlossen, dass ein Öffnen des Behälters im Wesentlichen unwiderruflich ist (d.h. nur mit erheblichem Aufwand wieder rückgängig zu machen ist). Ein typisches Beispiel hierfür sind Brechampullen z.B. aus Plastik oder Glas. Eine Brechampulle ist beispielsweise aus der EP 0 243 580 A1 oder der EP 0 350 772 A1 bekannt.

In einer weiteren bevorzugten Ausführungsform ist der erfindungsgemäße geschlossene Behälter ausgewählt aus:
- einer Ampulle insbesondere aus Kunststoff oder Glas, vorzugsweise eine Brechampulle insbesondere aus Kunststoff oder Glas (jeder Behälter, der einen Verschluss aufweist, welcher aufgebrochen werden muss, fällt im Kontext der vorliegenden Erfindung unter den Begriff der "Brechampulle")
- einer Durchstechflasche (auch als "Vial" bezeichnet; vgl. z.B. die WO 2006/072440 A1), bevorzugt versiegelt mit einer Schutzkappe,
- einem Kompartiment eines Misch- oder Rekonstitutionssystems, vorzugsweise wobei das Misch- oder Rekonstitutionssystem ein weiteres Kompartiment mit Lösungsmittel zur Rekonstitution des Lyophilisats aufweist,
- einem Infusionsbeutel oder einer Infusionsflasche (z.B. mit Luer-Lock oder Gummistopfen, gegebenenfalls mit Graduierung) und
- einer Spritze, insbesondere einer Fertigspritze. Der Behälter kann aus einem transparenten oder intransparenten Material gefertigt sein. Vorzugsweise stellt der Behälter einen Lichtschutz für das Lyophilisat dar (insbesondere im UV/VIS-Bereich). Der Behälter kann im Wesentlichen lichtundurchlässig bzw. lichtundurchlässig sein (insbesondere im UV/VIS-Bereich). Bevorzugt ist der Behälter ein Einwegbehälter bzw. zum einmaligen Gebrauch.

Misch- oder Rekonstitutionssysteme für Lyophilisate sind beispielsweise aus der WO 2016/107811 A1 oder der EP 2 962 676 A1 bekannt, und sind z.B. unter den Marken nextaro^{®} (sfm medical devices GmbH, Deutschland) oder FillChoice^{®} room (Haemopharm Healthcare, PAOLO GOBBI FRATTINI S.r.l., Italien) gewerblich erhältlich. Es gibt Systeme, die beispielsweise mit handelsüblichen Durchstechflaschen kompatibel und auf diese aufsetzbar sind (so dass der geschlossene Behälter, der das Lyophilisat beinhaltet, im Stadium der Endverpackung nicht integraler Teil des Systems ist - sehr wohl kann dieses System aber mit dem Behälter mitgeliefert werden), aber auch Systeme, die das Lyophilisat und gegebenenfalls das Lösungsmittel bereits beinhalten (mit anderen Worten stellt dasjenige Kompartiment (oder diejenige Kammer) des Systems, welches das Lyophilisat beinhaltet und den frühzeitigen Kontakt mit dem Lösungsmittel verhindert, den geschlossenen Behälter dar). Manche dieser Misch- oder Rekonstitutionssysteme werden auch als Überleitgeräte bezeichnet.

Zweckmäßigerweise weist der geschlossene Behälter, insbesondere, wenn er eine Durchstechflasche oder ein Infusionsbeutel ist, ein unversehrtes Septum beispielsweise aus Gummi auf, bzw. sind alle Septa, die der Behälter aufweist, unversehrt.

In einer weiteren bevorzugten Ausführungsform der vorliegenden Erfindung ist das pharmazeutisch annehmbare Salz von Fosfomycin ausgewählt aus Fosfomycin-Dinatriumsalz, Fosfomycin-Mononatriumsalz, einem Fosfomycin-Kaliumsalz, einem Fosfomycin-Lithiumsalz, Fosfomycin-Magnesiumsalz und Fosfomycin-Calciumsalz. Besonders bevorzugt sind die Natriumsalze, insbesondere Fosfomycin-Dinatriumsalz.

In einer weiteren bevorzugten Ausführungsform ist die pharmazeutisch annehmbare Säure eine schwache Säure oder eine organische Säure, vorzugsweise eine schwache organische Säure. Schwache Säuren sind insbesondere wegen ihrer Pufferwirkung bevorzugt. Insbesondere hat die, bevorzugt organische, Säure bei 25°C einen pKₛ-Wert von 2 bis 9, bevorzugt von 2,5 bis 8, mehr bevorzugt von 3 bis 7, noch mehr bevorzugt von 3,5 bis 6,5, insbesondere von 4 bis 6, vorzugsweise sind alle pKₛ-Werte der Säure (sofern die Säure mehrere pKₛ-Werte aufweist) unabhängig voneinander in einem dieser Intervalle. Zweckmäßigerweise ist die pharmazeutisch annehmbare Säure venenverträglich.

In einer weiteren, besonders bevorzugten Ausführungsform der vorliegenden Erfindung ist die pharmazeutisch annehmbare Säure ausgewählt aus Bernsteinsäure, Weinsäure, Milchsäure, Apfelsäure, Zitronensäure, Kohlensäure, Aminosäuren, Essigsäure und Phosphorsäure. Bernsteinsäure ist, vor allem für die intravenöse Verabreichung, ganz besonders bevorzugt.

Zweckmäßigerweise sind in der wässrigen Lösung (vor der Lyophilisierung) im Wesentlichen nur gelöst: ein pharmazeutisch annehmbares Salz von Fosfomycin und eine pharmazeutisch annehmbare Säure, gegebenenfalls mit Glucose oder Fructose (z.B. in einer Konzentration von 1% bis 5% w/v).

In einer weiteren bevorzugten Ausführungsform enthält die wässrige Lösung (vor der Lyophilisierung) als Antibiotikum nur Fosfomycin. Vorzugsweise enthält die wässrige Lösung überhaupt nur Fosfomycin als Wirkstoff (mit der pharmazeutisch annehmbaren Säure als Hilfstoff), gegebenenfalls mit weiteren Hilfsstoffen, insbesondere Glucose oder Fructose (z.B. in einer Konzentration von 1% bis 5% w/v).

In einer höchst bevorzugten Ausführungsform der vorliegenden Erfindung sind in der wässrigen Lösung (vor der Lyophilisierung) im Wesentlichen nur Fosfomycin-Dinatriumsalz und Bernsteinsäure gelöst, gegebenenfalls mit Glucose oder Fructose (z.B. in einer Konzentration von 1% bis 5% w/v).

In einer weiteren bevorzugten Ausführungsform weist die wässrige Lösung (vor der Lyophilisierung) oder die aus dem Lyophilisat hergestellte Lösung zur parenteralen Verabreichung (bevorzugt bei 25°C) einen pH-Wert von weniger als 10,0, bevorzugt von weniger als 9,0, mehr bevorzugt von weniger als 8,5, noch mehr bevorzugt von weniger als 8,0, insbesondere von weniger als 7,75 oder gar von höchstens 7,5 auf.

In einer weiteren bevorzugten Ausführungsform weist die wässrige Lösung (vor der Lyophilisierung) oder die aus dem Lyophilisat hergestellte Lösung zur parenteralen Verabreichung (bevorzugt bei 25°C) einen pH-Wert von 3 bis 9, bevorzugt von 4 bis 8,5, mehr bevorzugt von 5 bis 8,5, insbesondere von 6 bis weniger als 8,0, auf.

In einer weiteren, besonders bevorzugten Ausführungsform weist die wässrige Lösung (vor der Lyophilisierung) oder die aus dem Lyophilisat hergestellte Lösung zur parenteralen Verabreichung (bevorzugt bei 25°C) einen pH-Wert von 6,4 bis 8,4, bevorzugt von mehr als 6,5 und weniger als 8,0, eher bevorzugt von 6,9 bis 7,9, mehr bevorzugt von 7,0 bis 7,8, noch mehr bevorzugt von 7,1 bis 7,7, insbesondere von 7,2 bis 7,6 oder gar von 7,3 bis 7,5, auf. Insbesondere diese Ausführungsform ist für Infusion oder Injektion bzw. die intravenöse Verabreichung geeignet, wobei pH-Werte, die möglichst nahe beim normalen pH-Wert des menschlichen Blutes (7,35-7,45) liegen, besonders zweckmäßig sind.

In einer weiteren, bevorzugten Ausführungsform hat die wässrige Lösung (vor der Lyophilisierung) oder die aus dem Lyophilisat hergestellte Lösung zur parenteralen Verabreichung (insbesondere wenn sie gebrauchsfertig zur intravenösen Verabreichung vorliegt) eine Osmolarität von höchstens 800 mosmol/L, bevorzugt höchstens 600 mosmol/L, mehr bevorzugt höchstens 500 mosmol/L, noch mehr bevorzugt höchstens 450 mosmol/L, insbesondere höchstens 400 mosmol/L oder gar höchstens 350 mosmol/L; zweckmäßigerweise bei einer gewissen Mindestosmolarität von z.B. mindestens 50 mosmol/L, mindestens 100 mosmol/L oder mindestens 200 mosmol/L. Ganz besonders bevorzugt ist die wässrige Lösung (vor der Lyophilisierung) oder die aus dem Lyophilisat hergestellte Lösung zur parenteralen Verabreichung Lösung isotonisch oder hypotonisch (im Bezug auf menschliches Blutplasma), zweckmäßigerweise bei einer gewissen Mindestosmolarität von z.B. mindestens 50 mosmol/L, mindestens 100 mosmol/L oder mindestens 200 mosmol/L.

Vorteilhafterweise enthält der geschlossene Behälter (im Lyophilisat) eine Fosfomycin-Gesamtdosis entsprechend 0,25 bis 15 Gramm Fosfomycin, bevorzugt 0,5 bis 10 Gramm Fosfomycin, mehr bevorzugt 0,75 bis 8 Gramm Fosfomycin, insbesondere 1 bis 4 Gramm Fosfomycin. Insbesondere enthält der geschlossene Behälter eine Einzeldosis. Vorzugsweise liegt das Volumen der Lösung nach Rekonstitution (insbesondere wenn sie gebrauchsfertig vorliegt) zwischen 1 ml und 100 ml / g Fosfomycin, bevorzugt zwischen 5 ml und 50 ml / g Fosfomycin, mehr bevorzugt zwischen 10 ml und 40 ml / g Fosfomycin, noch mehr bevorzugt zwischen 22,5 ml und 27,5 ml / g Fosfomycin, insbesondere bei im Wesentlichen 25 ml / g Fosfomycin.

In einer weiteren bevorzugten Ausführungsform ist die pharmazeutisch annehmbare Säure Bernsteinsäure und beträgt das Massenverhältnis von Bernsteinsäure zu Fosfomycin in der wässrigen Lösung (vor dem Lyophilisieren) bzw. im Lyophilisat im geschlossenen Behälter zwischen 30:1 und 50:1, insbesondere im Wesentlichen 40:1.

Zweckmäßigerweise ist der Behälter steril mit der Lösung befüllt worden und/oder der geschlossene Behälter mit dem Lyophilisat sterilisiert worden. Insbesondere entspricht der geschlossene Behälter bzw. das erfindungsgemäße Verfahren den am 15. Februar 2016 gültigen "EU Guidelines for Good Manufacturing Practice for Medicinal Products for Human and Veterinary Use", insbesondere Annex 1 in der Fassung "25 November 2008 (rev.)".

In einer weiteren bevorzugten Ausführungsform ist der Behälter gemäß Annex 1 der "EU Guidelines for Good Manufacturing Practice for Medicinal Products for Human and Veterinary Use" in der Fassung "25 November 2008 (rev.)" mit der wässrigen Lösung befüllt und geschlossen worden; bzw. das erfindungsgemäße Verfahren gemäß dieser Vorschrift durchgeführt worden.

Gemäß einer weiteren bevorzugten Ausführungsform umfasst das Verfahren den Schritt des Verpackens des geschlossenen Behälters in eine Verpackung; bevorzugt wobei der geschlossene Behälter gemeinsam mit einem weiteren Behälter mit Lösungsmittel zur Rekonstitution des Lyophilisats, und insbesondere auch mit einem Misch- oder Rekonstitutionssystem, verpackt wird. Die Verpackung kann unter anderem aus Karton, insbesondere bedrucktem Karton, gefertigt sein. Die Verpackung umfasst vorzugsweise eine Gebrauchsinformation. Vorzugsweise stellt die Verpackung einen Lichtschutz für das Lyophilisat bzw. den Behälter dar (insbesondere im UV/VIS-Bereich). Die Verpackung kann im Wesentlichen lichtundurchlässig bzw. lichtundurchlässig sein (insbesondere im UV/VIS-Bereich). Bevorzugt wahrt die Verpackung zusätzlich die Sterilität des geschlossenen Behälters (also auch seiner äußeren Oberfläche), z.B. wenn die Verpackung oder ein Teil davon als Plastikfolie ausgeführt ist.

Vorteilhafterweise wird die Verpackung versiegelt, unter anderem aus Gründen der Manipulationssicherheit bzw. auch um versehentliches Wiederverwenden zu verhindern. Der Ausdruck "versiegelt" bedeutet in diesem Kontext, dass ein Öffnen der Verpackung, das nach dem Versiegeln stattgefunden hat, nachweisbar, bevorzugt sogar mit freiem Auge sichtbar, ist, wobei die besagte Nachweisbarkeit bzw. Sichtbarkeit zumindest nicht ohne Aufwand bzw. ohne erheblichen Aufwand wieder rückgängig gemacht werden kann. Dem Fachmann sind Mittel zur Versiegelung von Verpackungen im Arzneimittelbereich bekannt, darunter fallen beispielsweise Abziehfolien, Faserrissetiketten und Void-Siegel. Vorzugsweise erfüllt die Versiegelung der Verpackung bzw. die Verpackung die Norm DIN EN 16679:2015-03.

Weitere Definitionen:
Hierin ist "parenteral" in seiner breitesten Bedeutung ("unter Umgehung des Darmes") auszulegen, d.h. es werden durch diesen Begriff alle Arten der Verabreichung außer der oralen oder der rektalen umfasst.
Vorzugsweise ist das Wasser in der wässrigen Lösung bzw. das Lösungsmittel Wasser zu Injektionszwecken, insbesondere gemäß Ph. Eur. (8. Auflage).
Die Erfindung wird ferner durch das folgende Beispiel, auf welches es selbstverständlich nicht eingeschränkt ist, veranschaulicht.

### Beispiel 1 - Herstellung von Fosfomycin 1g i.v.

In 250 L Wasser für Injektionszwecke gemäß Ph. Eur. (8. Auflage) werden 10000 g Fosfomycin und 3200 g Bernsteinsäure unter sterilen Bedingungen gelöst. Diese Lösung wird unter Sterilbedingungen in 10000 30ml-Durchstechflaschen abgefüllt. Danach findet die Lyophilisation statt. Die Durchstechflaschen mit Lyophilisat werden verschlossen und zu je 10 Stück in einen Karton verpackt.

Vor der intravenösen Anwendung beim Patienten wird durch eine Nadel 25 ml Wasser für Injektionszwecke gemäß Ph. Eur. (8. Auflage) in die Durchstechflasche eingebracht, um das Lyophilisat zu rekonstituieren.

### Beispiel 2 - Herstellung von Fosfomycin 8g i.v.

In 250 L Wasser für Injektionszwecke gemäß Ph. Eur. (8. Auflage) werden 100000 g Fosfomycin und 25600 g Bernsteinsäure unter sterilen Bedingungen gelöst. Diese Lösung wird unter Sterilbedingungen in 10000 30ml-Durchstechflaschen abgefüllt. Danach findet die Lyophilisation statt. Die Durchstechflaschen mit Lyophilisat werden verschlossen und einzeln, jeweils gemeinsam mit einem Lösungsmittelbehälter (Wasser für Injektionszwecke gemäß Ph. Eur. (8. Auflage)), steril abgepackt.

Vor der intravenösen Anwendung beim Patienten wird durch eine Nadel 25 ml des Lösungsmittels in die Durchstechflasche eingebracht und danach mit weiterem Lösungsmittel auf ein Gesamtvolumen von 200 ml verdünnt, um das Lyophilisat zu rekonstituieren.

## Patentansprüche

1. Verfahren zur Herstellung eines geschlossenen Behälters, wobei der Behälter ein Lyophilisat zur Rekonstitution zu einer Lösung zur parenteralen Verabreichung beinhaltet, umfassend die folgenden Schritte:
- Herstellen einer wässrigen Lösung, wobei in der Lösung zumindest Fosfomycin bzw. ein pharmazeutisch annehmbares Salz davon, vorzugsweise als einziger Wirkstoff, und eine pharmazeutisch annehmbare Säure gelöst sind,
- Befüllen zumindest eines Behälters mit der wässrigen Lösung,
- Lyophilisieren der wässrigen Lösung im Behälter und
- Verschließen des Behälters, wodurch der geschlossene Behälter erhalten wird.

2. Verfahren nach Anspruch 1, wobei der Behälter versiegelt wird, vorzugsweise wobei der Behälter in einer Weise geschlossen wird, dass ein Öffnen des Behälters im Wesentlichen unwiderruflich ist.

3. Verfahren gemäß einem der Ansprüche 1 bis 2, wobei der geschlossene Behälter ausgewählt ist aus:
- einer Ampulle insbesondere aus Kunststoff oder Glas, vorzugsweise eine Brechampulle insbesondere aus Kunststoff oder Glas,
- einer Durchstechflasche, bevorzugt versiegelt mit einer Schutzkappe,
- einem Kompartiment eines Misch- oder Rekonstitutionssystems, vorzugsweise wobei das Misch- oder Rekonstitutionssystem ein weiteres Kompartiment mit Lösungsmittel zur Rekonstitution des Lyophilisats aufweist,
- einem Infusionsbeutel und
- einer Spritze, insbesondere einer Fertigspritze.

4. Verfahren gemäß einem der Ansprüche 1 bis 3, wobei der geschlossene Behälter, der vorzugsweise eine Durchstechflasche oder ein Infusionsbeutel ist, ein unversehrtes Septum, beispielsweise aus Gummi, aufweist.

5. Verfahren gemäß einem der Ansprüche 1 bis 4, wobei der Behälter einen Lichtschutz für das Lyophilisat darstellt, insbesondere im Wesentlichen lichtundurchlässig ist.

6. Verfahren gemäß einem der Ansprüche 1 bis 5, wobei das pharmazeutisch annehmbare Salz von Fosfomycin ausgewählt ist aus Fosfomycin-Dinatriumsalz, Fosfomycin-Mononatriumsalz, einem Fosfomycin-Kaliumsalz, einem Fosfomycin-Lithiumsalz, Fosfomycin-Magnesiumsalz, und Fosfomycin-Calciumsalz.

7. Verfahren gemäß einem der Ansprüche 1 bis 8, wobei die pharmazeutisch annehmbare Säure bei 25°C einen pKₛ-Wert von 2 bis 9 aufweist, wobei die Säure bevorzugt ausgewählt ist aus Bernsteinsäure, Weinsäure, Milchsäure, Apfelsäure, Zitronensäure, Kohlensäure, Aminosäuren, Essigsäure und Phosphorsäure.

8. Verfahren gemäß einem der Ansprüche 1 bis 7, wobei die Lösung zur parenteralen Verabreichung nach Rekonstitution mit Wasser oder Kochsalzlösung einen pH-Wert von 6,4 bis 8,4, bevorzugt von mehr als 6,5 und weniger als 8,0, eher bevorzugt von 6,9 bis 7,9, mehr bevorzugt von 7,0 bis 7,8, noch mehr bevorzugt von 7,1 bis 7,7, insbesondere von 7,2 bis 7,6 oder gar von 7,3 bis 7,5, aufweist; oder
wobei die Lösung einen pH-Wert von weniger als 10,0, bevorzugt von weniger als 9,0, mehr bevorzugt von weniger als 8,5, noch mehr bevorzugt von weniger als 8,0, insbesondere von weniger als 7,75 oder gar von höchstens 7,5 aufweist.

9. Verfahren gemäß einem der Ansprüche 1 bis 8, wobei die pharmazeutisch annehmbare Säure Bernsteinsäure ist und das Massenverhältnis von Bernsteinsäure zu Fosfomycin im Lyophilisat zwischen 30:1 und 50:1 liegt, insbesondere bei im Wesentlichen 40:1.

10. Verfahren gemäß einem der Ansprüche 1 bis 9, wobei das Verfahren den am 15. Februar 2016 gültigen "EU Guidelines for Good Manufacturing Practice for Medicinal Products for Human and Veterinary Use", insbesondere Annex 1 in der Fassung "25 November 2008 (rev.)", entspricht; und/oder
wobei der Behälter gemäß Annex 1 der "EU Guidelines for Good Manufacturing Practice for Medicinal Products for Human and Veterinary Use" in der Fassung "25 November 2008 (rev.)" mit der Lösung befüllt und geschlossen wird.

11. Verfahren gemäß einem der Ansprüche 1 bis 10, ferner umfassend den Schritt des Verpackens des geschlossenen Behälters in eine Verpackung, vorzugsweise mit einer Gebrauchsinformation;
bevorzugt wobei die Verpackung einen Lichtschutz für das Lyophilisat bzw. den geschlossenen Behälter darstellt, insbesondere im Wesentlichen lichtundurchlässig ist; und/oder
bevorzugt wobei der geschlossene Behälter gemeinsam mit einem weiterem Behälter mit Lösungsmittel zur Rekonstitution des Lyophilisats, und insbesondere auch mit einem Misch- oder Rekonstitutionssystem, verpackt wird.

12. Geschlossener Behälter, wobei der Behälter ein Lyophilisat zur Rekonstitution zu einer Lösung zur parenteralen Verabreichung beinhaltet, wobei das Lyophilisat Fosfomycin bzw. ein pharmazeutisch annehmbares Salz davon und eine pharmazeutisch annehmbare Säure bzw. ein pharmazeutisch annehmbares Salz davon umfasst.

13. Geschlossener Behälter nach Anspruch 12, erhältlich nach dem Verfahren gemäß einem der Ansprüche 1 bis 11.

14. Set, umfassend den geschlossenen Behälter nach Anspruch 12 oder 13, ferner umfassend einen weiteren Behälter mit Lösungsmittel zur Rekonstitution des Lyophilisats und vorzugsweise ein Misch- oder Rekonstitutionssystem.

15. Verfahren zur Herstellung einer Lösung zur parenteralen Verabreichung, umfassend das Öffnen des geschlossenen Behälters gemäß Anspruch 12 oder 13 oder das Öffnen des geschlossenen Behälters des Sets gemäß Anspruch 14, und das Rekonstituieren des Lyophilisats im Behälter zu der besagten Lösung.
